Europäisches Patentamt

European Patent Office

Office européen des brevets

(11) Publication number: **0 261 534**
**A2**

## EUROPEAN PATENT APPLICATION

(21) Application number: 87113378.1

(22) Date of filing: 14.09.87

(51) Int. Cl.⁴: **C12N 15/00** , C12N 1/18 ,
C12N 1/38 , //C12N9/38

(30) Priority: 23.09.86 US 910871

(43) Date of publication of application:
30.03.88 Bulletin 88/13

(84) Designated Contracting States:
AT BE CH DE FR GB IT LI LU NL SE

(71) Applicant: **MILES LABORATORIES, INC.**
**1127 Myrtle Street**
**Elkhart Indiana 46514(US)**

(72) Inventor: **Das, Rathindra C.**
**2321 Kenilworth Drive**
**Elkhart Indiana 46514(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) Improved gene expression in yeast.

(57) DNA constructs are provided which direct high expression of structural genes in yeast. Said DNA constructs utilize the yeast mating factor $\alpha$-1 prepro-coding sequence tandemly linked to a 103 base pair region of the SUC2 gene encoding the 34 amino-terminal amino acids of the invertase gene, and a structural gene encoding a polypeptide of choice in reading frame therewith.

EP 0 261 534 A2

# IMPROVED GENE EXPRESSION IN YEAST

## Background of the Invention and Pertinent Art

Molecular biological techniques have enabled the production of relatively large quantities of polypeptides of diverse origin in microbial hosts. Early efforts at gene cloning and expression focused principally on the use of procaryotic hosts, particularly E. coli due to the extent of characterization and understanding of the genetics of the organism. However, the use of E. coli as a host has several disadvantages. For example, because E. coli is a nominal inhabitant of the human intestinal tract and is potentially pathogenic, large scale production of products derived from cloned DNA in E. coli may present certain health risks. Even in the case of nonpathogenic strains, E. coli endotoxins are produced which could contaminate the particular polypeptide produced by the organism. E. coli also retains extracellular enzymes in the periplasmic space thereby making isolation and purification of the exogenous polypeptide difficult.

In an attempt to overcome these difficulties, the gram-positive organism Bacillus subtilis was investigated as a possible host for cloning vectors. Although the technology for cloning in B. subtilis was not as well developed as for E. coli, certain advantages were clear. First, B. subtilis is non-pathogenic and does not produce potentially contaminating endotoxins. Significantly, B . subtilis excretes into the culture medium the polypeptides produced therein thus making isolation and purification easier. Nonetheless, various disadvantages were also attendant. Among them is plasmid instability once transformed into the host making it extremely difficult to maintain plasmid replication over many culture transfers. Also heterologous DNA is not well maintained in B. subtilis such that the cloned DNA is frequently unexpectedly lost. Further, very few efficient regulatory systems are available for B. subtilis, making its use as an expression vector problematical.

With the evolution of the technology, lower eucaryotic hosts such as yeasts were examined. The utilization of yeasts (particularly Saccharomyces cerevisiae) as a host organism for cloning vectors carries several distinct advantages. For example, being a eucaryote, yeast share a greater degree of similarlity with higher eucaryotic organisms as possessing the cellular machinery necessary to carry out various post-translational modifications of the polypeptide produced (e.g., glycosylation) thus enabling production of the mature form of the polypeptide. Further, fermentation techniques for yeast are well established thus making commercial scale production of a chosen polypeptide in yeast feasible. Significantly, yeast have also been reported to secrete endogenous polypeptides either into the periplasmic space or into the cellular medium (Schekman et al, 1982). Those yeast polypeptides reportedly secreted into the cellular growth medium include the yeast pheromones mating factor α and a, pheromone peptidase and "killer toxin". Among those yeast polypeptides transported into the periplasmic space are invertase, L-asparaginase and the repressible and constitutive forms of acid phosphatase. Certain yeast polypeptides are known to be localized in the periplasmic space and also secreted out of the cell. These include α-galactosidase, exo-1,3-β-glucanase and endo-1,3-β-glucanase. Thus, this information coupled with the fact that yeast are capable of intracellular processing of endogenous polypeptides in a manner analogous to that of mammalian systems has led to increased interest in the genetics of expression and secretion of both endogenous and exogenous polypeptides in yeast.

For example, Kurjan et al (U.S. Patent No. 4,546,082) describe the cloning of the gene for the polypeptide α-factor which is a secreted polypeptide from the yeast Saccharomyces cerevisiae. Mating in yeast appears to be facilitated by such pheromones ("mating factors") that cause arrest of cells of opposite mating type in the G1 phase of the cell division cycle. Yeast α-cells produce the α-factor which is in two forms, 13 and 12 L-amino acids in length, the latter lacking the amino-terminal Trp residue of the peptide. a-factor is produced by yeast a-cells and is comprised of 11 amino acids in length and has two forms which differ in the sixth residue (Leu or Val). Synthesis of α-factor is said to require cellular RNA and protein synthesis. Thus, based on these observations and by analogy with mammalian peptide hormones, Kurjan et al state that there is support for the proposal that the yeast mating factors are derived by proteolytic processing from a larger precursor molecule. Thus, cloning of the α-factor structural gene was accomplished and the genetic structure of the precursor was determined. Based on this work it was found that the α-factor precursor gene comprises four sequences coding for α-factor, each of which is separated by a spacer nucleotide region. It is believed that the spacer regions provide peptides which are substrates for the yeast proteolytic processing system. Thus, by deleting the α-factor coding sequences in the α-factor precursor and by inserting genes encoding other "useful" proteins between the spacer coding regions, or by fusing to these regions, the resultant fusion polypeptide may be expressed, subjected to the yeast proteolytic processing system and ultimately be secreted from the cell.

Since the work of Kurjan et al, others have attempted to elucidate the mechanism of α-factor directed expression and secretion in yeast. For example, Emr, et al (1983) describe an in-frame fusion of the structural genes encoding α-factor and invertase (β-D-fructofuranoside fructohydrolase). Sites suggested as necessary for intracellular proteolytic processing of the α-factor precursor were retained and it was found that the chimeric gene directed the synthesis of a high level of active invertase which was secreted within the periplasmic space. Similarly, Brake et al (1984) describe the construction of plasmids containing hybrid genes in which the sequence encoding mature, human epidermal growth factor was joined to various sequences encoding the leader region (prepro-segment) of the precursor of the yeast α-factor. Said plasmids were used to transform Saccharomyces cerevisiae cells, which cells accurately processed the hybrid protein and efficiently secreted mature, biologically active human epidermal growth factor into the medium. Still another gene construct utilizing the α-factor promoter was reported by Bitter et al (1984). Fusions between the α-factor structural gene and each of β-endorphin and α-interferon were made while maintaining the proteolytic cleavage sites involved in α-factor maturation. The α-factor promoter expressed the gene fusions in Saccharomyces cerevisiae and resulted in the efficient secretion of the heterologous proteins into the culture medium. Finally, in an attempt to define the genetic information needed for the transport out of the cells of secretory proteins in yeast, Emr et al (1984) constructed a series of fusions between the yeast SUC2 gene (encoding invertase) and the E. coli lacZ gene (encoding the cytoplasmic enzyme β-galactosidase). The expressed invertase-β-galactosidase hybrid proteins were, however, not secreted into the nutrient medium but rather remained trapped in the endoplasmic reticulum.

Given the above-described background information, it remains clear that eucaryotic hosts such as the yeast represent viable vehicles for the expresion and possible secretion out of the cell of heterologous polypeptides of biochemical significance. However, before the promise of benefits flowing from molecular biological advances are realized on a commercial scale, it is clearly necessary to continue to provide advances which will allow the production of greater amounts of the polypeptide of interest by, for example improving expression levels, providing for accurate post-translational proteolytic processing and ultimate secretion out of the cell. The present invention is directed to such an improvement by providing a gene construct of the prepro-coding sequence of yeast mating factor α-1 tandemly linked to a 103 base pair fragment of the invertase signal sequence. Said gene construct is capable of directing extremely high levels of expression of a structural gene encoding a polypeptide of choice when said structural gene is placed immediately downstream of said invertase fragment.

## Summary of the Invention

The present invention is directed to DNA constructs capable of directing a high level of expression of a structural gene in yeast. Said DNA constructs comprise the yeast mating factor α-1 prepro-coding sequence tandemly linked to a 103 base pair region of the yeast SUC2 gene encoding the 34 amino-terminal amino acids of the invertase gene, and a structural gene encoding a polypeptide of choice in reading frame therewith.

Also disclosed and claimed is a method for increasing the level of expression of a gene encoding a polypeptide of choice by culturing a yeast cell containing said gene in the presence of one or more chloride salts of alkali metals or alkaline-earth metals.

## Brief Description of the Drawings

Figure 1 is the nucleotide sequence for the yeast mating factor α-1, including the prepro-coding sequence.

Figure 2 is the nucleotide sequence encoding the 34 amino-terminal amino acids of the yeast SUC2 gene.

Figure 3 is a diagrammatic representation of the construction of plasmid pJS12.

## Detailed Description of the Invention

As alluded to earlier, the DNA constructs of the present invention represent a tripartite gene capable of directing high levels of expression of a structural gene encoding a polypeptide of choice. Said tripartite gene includes the prepro-coding sequence of yeast mating factor α-1 fused in-frame to a 103 base pair segment of the yeast SUC2 gene fragment. Fused immediately downstream of the SUC2 gene fragment is the structural gene encoding the polypeptide of choice.

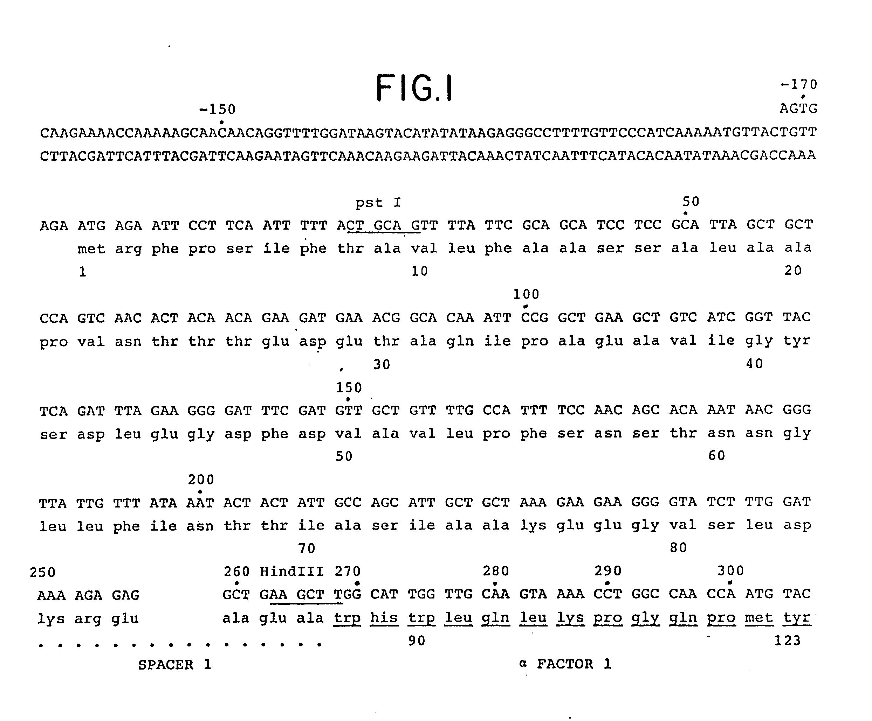

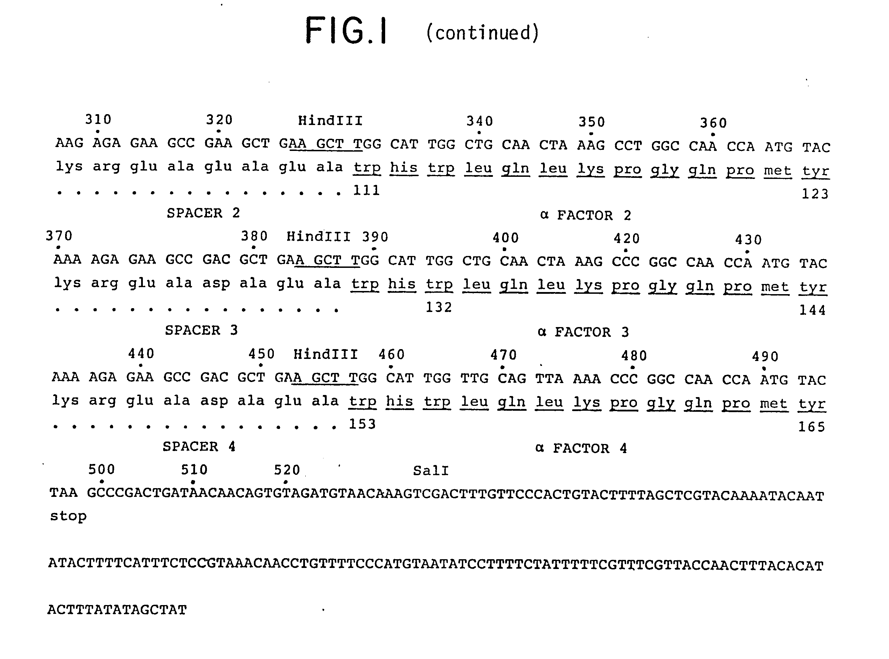

The prepro-coding sequence of yeast mating factor α-1 as used herein means that segment from base 1 through the HindIII cleavage site at base 263 (i.e., GA'A) of said gene. This is graphically represented in Figure 1. Within this prepro-coding sequence is an ATG translation initiation codon as well as a putative signal peptide cleavage site at amino acid 20 (i.e., ala-x). The functional native prepro-coding sequence extends from base 1 through base 249. The region from base 250 through base 263 represents a portion of the first of the spacer nucleotide regions immediately preceding each of the four coding regions encoding the α-factor precursor molecule. As used herein, the term prepro-coding sequence is meant to include said portion of said spacer nucleotide region from base 250 through base 263.

The prepro-coding sequence of yeast mating factor α-1 may be obtained from the cloned α-factor precursor gene such as described by Kurjan et al, 1982 as follows. A clone bank containing random genomic fragments of yeast DNA is inserted into the high copy number plasmid YEp13. This plasmid contains the origin of replication of the yeast 2μ replicon and is present in 30-50 copies per cell. A yeast strain (such as a mat alpha-2 leu 2) is then transformed with plasmid DNA from the clone bank and Leu+ transformants are selected, pooled and plated on a selective medium lacking leucine. The colonies capable of secreting α-factor are screened by halo formation on a lawn of a cells. The gene responsible for halo formation is located on a 1.7 kilobase (kb) EcoR1 fragment of a producing plasmid. Said EcoR1 fragment is inactivated by cleavage with HindIII suggesting that the HindIII sites may be within the gene responsible for α-factor production. The results of Maxam-Gilbert sequencing shown in Figure 1 describe a HindIII site at base 263 making cleavage and isolation of the prepro-coding sequence from the remainder of the coding region for the α-factor precursor molecule convenient and conventional in the art.

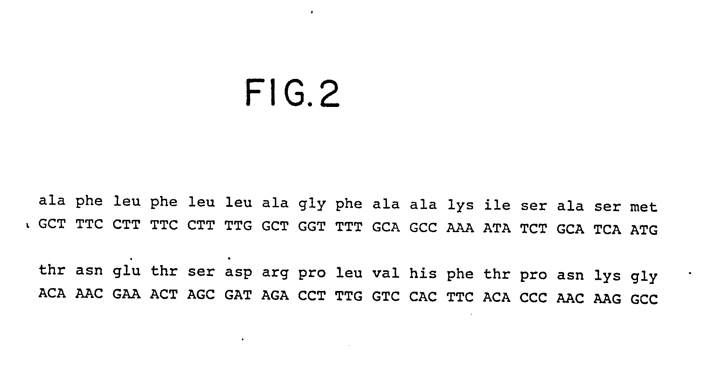

To the prepro-coding sequence for yeast mating factor α-1 is fused in-frame a 103 base pair segment of the yeast SUC2 gene. This SUC2 gene fragment encodes the 34 amino-terminal amino acids of the enzyme invertase which includes 15 of the 19 signal sequence amino acids and one potential glycosylation site (asn-X-thr). The nucleotide sequence for this fragment is shown in Figure 2. The SUC2 gene has been cloned from a YEp13 yeast library and its entire nucleotide sequence has been determined. See, Carlson et al (1982) and Taussig et al (1983). Based on this information, the SUC2 gene fragment as used in the present invention is readily obtained from the cloned gene by Bal31 restriction enzyme digestion of the amino-terminal segment of the cloned SUC2 DNA. This fragment may then be ligated by standard techniques to the yeast mating factor α-1 prepro-coding sequence.

As will be seen below in control studies, the high levels of expression of genes encoding the polypeptide of choice is directly attributable to the presence of the SUC2 gene fragment in the tripartite gene constructs of the present invention. It will be readily evident to the skilled artisan to prepare constructs utilizing only a portion of the 103 nucleotide fragment disclosed herein or to prepare constructs using a greater number of the amino-terminal amino acids of the SUC2 gene and still obtain a construct capable of directing high levels of expression of the gene of choice. While a specific 103 nucleotide fragment encoding a portion of the amino terminus of the SUC2 gene is described herein, contemplated equivalents of this fragment include any portions thereof or longer segments of said amino terminus which retain the ability to direct high levels of expression of the gene encoding the polypeptide of choice.

The structural gene encoding the polypeptide of choice is fused immediately downstream of the SUC2 gene fragment. Such fusions are accomplished by standard ligation techniques as disclosed, for example, in Maniatis et al (1982). Said structural gene will typically, though not necessarily, be heterologous to yeast and may encode a procaryotic or eucaryotic polypeptide such as growth hormones, somatostatin, epidermal growth factor, luteinizing hormone, thyroid stimulating hormone, relaxin, secretin, oxytocin, insulin, vasopressin, renin, calcitonin, hematopoietic factors such as erythropoietin, various lymphokines, immunoglobulins, albumins, interferons, endorphins, calf chymosin, xylose isomerase, α-amylase, β-galactosidase and the like.

The tripartite gene thus formed is then incorporated into a transformation vector so as to stably introduce the resulting construct into the yeast host. This may be accomplished, for example, by either cleaving said transformation vector with a restriction enzyme that produces cohesive termini complementary to the tripartite gene or by modifying the termini of either the vector or tripartite gene so that they are complementary, allowing the complementary sequences to anneal and then ligating the resulting transformation vector. Suitable transformation vectors include those capable of the stable incorporation of the tripartite gene in yeast and the ultimate expression of the structural gene encoding the polypeptide of choice. Such vectors include, for example, plasmids pYαE, pYcαE, pJY6, YEp13, YEp24, YCp19 (available from the American Type Culture Collection), and the like.

Said transformation vector is then incorporated into the host yeast cell of choice. The specific yeast selected is not critical to the invention and may be any of those yeast commonly used as hosts for the expression of cloned genes such as various species of Saccharomyces and mutants thereof, particularly Saccharomyces cerevisiae and mutants thereof. Transformation of said yeast host with the vector incorporating the tripartite gene may be affected by conventional methodologies such as, for example, those described by Ito et al (1983). The resulting transformants are then cultured under conditions facilitating expression of the structural gene encoding the polypeptide of choice, said conditions similarly being conventional in the art.

However, while the general culture conditions chosen are conventional, it has been found that the addition of one or more chloride salts of alkali metals or alkaline-earth metals to the culture medium increases gene expression to a level significantly higher than otherwise achieved in the absence of said chloride salts. Such salts include mono-and divalent salts such as sodium chloride, potassium chloride, calcuim chloride and the like. Preferably, the salt will be an alkali metal chloride and most preferably will be sodium chloride or potassium chloride. The chloride salt is present in the culture medium in an amount sufficient to increase the level of expression of the structural gene encoding the polypeptide of choice from the tripartite gene without otherwise having detrimental effects on the cultured cells. For sodium chloride and potassium chloride, this amount represents a concentration in the culture medium of preferably from about 100 millimolar (mM) to about 1 molar (M), more preferably from about 200 mM to about 800 mM. Specific optimal amounts of any such salts may be readily ascertained by conventional methodologies.

The resulting expression product when secreted into the culture medium may be harvested directly from said medium or, alternatively, when said expression product is maintained intracellularly (as in the periplasmic space, for example) the cells may be lysed by treatment with enzyme (e.g., zymolase) and the product harvested by centrifugation, for example. The isolated polypeptide may be subsequently purified by conventional techniques including filtration, chromatography, electrophoresis, dialysis, solvent extraction and the like.

The following examples are provided as a means of illustrating the present invention and should not be construed as a limitation thereon.

Example 1

a) Construction of a Tripartite Gene Coding for β-galactosidase

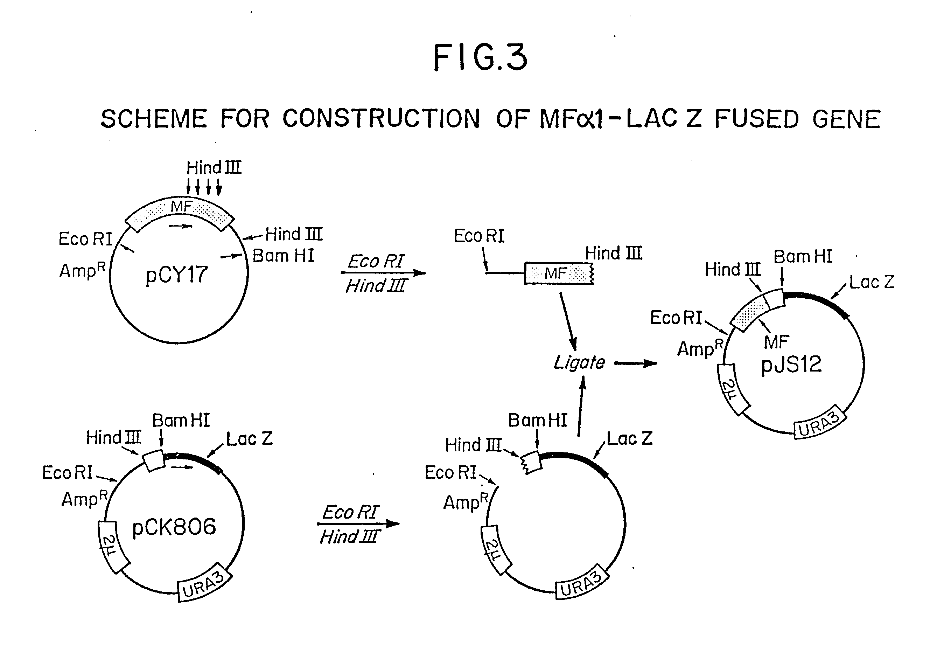

A tripartite gene encoding the enzyme β-galactosidase and present on plasmid pJS12 was prepared as graphically represented in Figure 3.

Plasmid pCY17, a bacterial plasmid containing the yeast mating factor α-1 structural gene (and prepro-coding sequence) is prepared as described by Kurjan et al, 1982 and U.S. Patent 4,546,082. Said plasmid was digested with restriction endonucleases EcoR1 and HindIII to render an 1100 base pair (bp) fragment extending from the EcoR1 site (950 bp upstream from the AUG initiation codon for the yeast mating factor α-1 coding region) to the HindIII site located immediately downstream of the prepro-coding sequence. This fragment, which contains the yeast mating factor α-1 prepro-coding sequence was isolated by chromatography and electroelution. Simultaneously, the yeast cloning vehicle pCK806 containing a SUC2-lacZ fusion is prepared as described by Emr et al, 1984. Said cloning vehicle was EcoR1 and HindIII digested and agarose gel purified to yield an 8.5 kb fragment. Said fragment contains the yeast 2μ replicon, URA3 gene and pBR322 sequences which encode the E. coli lacZ structural gene coding for β-galactosidase (absent the coding sequence for the eight amino-terminal amino acids thereof), said lacZ structural gene fused in-frame with the 103 base pair fragment of the yeast SUC2 gene (including a BamH1 linker at the 5′ end, as noted in Figure 3.

The previously isolated 1100 bp EcoR1 to HindIII fragment from plasmid pCY17 was then ligated into the 8.5 kb EcoR1 to HindIII fragment generated from plasmid pCK806, thereby generating plasmid pJS12 containing the tripartite gene including the prepro-coding sequence of yeast mating factor α-1 fused to a 103 base pair segment of the yeast SUC2 gene to which is fused the E. coli lacZ structural gene encoding the enzyme β-galactosidase. Plasmid pJS12 has been deposited with the American Type Culture Collection and has been given acession number 67210.

b) Transformation of Yeast with Plasmid pJS12 and Culturing of the Host Cells

The yeast Saccharomyces cerevisiae JRY188 (MATα, sir3-8$^{ts}$, ura3-52, leu2, trp, his4, rmel) were transformed with plasmid pJS12 by a slight modification of the method of Ito, et al (1983) as follows. 10 milliliters (ml) of a fresh logarithmic phase culture (2 x 10$^7$ cells/ml) was centrifuged at 7000 revolutions per minute (rpm) at room temperature for 10 minutes. The resulting pellet was washed once with a buffer containing 10 mM Tris HCl, pH 7.5 (also containing 1 mM EDTA and 100 mM lithium acetate) and was then suspended in 0.1 ml of the same buffer followed by incubation for 60 minutes at 30°C. Plasmid DNA (i.e., pJS12) was added to the yeast cells to a final concentration of from 1 to 10 microgram ($\mu$g) in the presence of 50 $\mu$g salmon sperm DNA and the cells were then incubated for 30 minutes at 30°C. To this was added 0.7 ml of a mixture of 50% polyethylene glycol 3350 (Sigma Chemical Company, St. Louis, Missouri USA), 1 M lithium acetate and 100 mM Tris HCl (pH 7.5, containing 10 mM EDTA) in a ratio of 8:1:1. The resultant mixture was vortexed briefly and incubated for 60 minutes at 30°C. The cells were then heat shocked for 5 minutes at 42°C, washed 2 times with 0.5 ml of TE buffer (i.e., 10 mM Tris HCl, pH 7.5 containing 1 mM EDTA) and then resuspended in 0.1 ml of TE buffer. The cells were then plated on an agar plate containing yeast minimal medium (i.e., 0.67% Difco yeast nitrogen base without amino acids, containing 2% glucose) supplemented with histidine, tryptophan and leucine. Plasmid-containing JRY188 cells were selected and grown at 37°C in a shaker flask at 300 rpm. Saturated cultures were then diluted 1:100 in fresh medium (as described above) and grown at 25°C up to the desired optical density ($A_{600}$) units.

c) $\beta$-galoctosidase Isolation and Assay

An aliquot of the above cells were separated from the growth medium by centrifugation at 7000 rpm. The cells were mixed with a buffer containing 60 mM $Na_2HPO_4 \bullet 7H_2O$, 40 mM $NaH_2PO_4 \bullet H_2O$, 10 mM KCl, 1 mM $MgSO_4 \bullet 7H_2O$ and 125 mM dithiothreitol to a final total volume of 1 ml. The cells were made permeable by the addition of 3 drops of chloroform followed by vigorous vortexing for 2 seconds. The resulting mixture was incubated for 5 minutes at 28°C.

$\beta$-galactosidase activity was determined as described by Miller, J. (1972). 0.2 ml of O-nitrophenyl-$\beta$-galoctoside (4 mg/ml) was added to the above incubation mixture. When the solution turned light yellow, the reaction was stopped by the addition of 0.5 ml of 1 M $Na_2CO_3$ and the exact duration of the reaction was noted. The cells were spun down at 10,000 rpm and the optical density (OD) of the clear supernatant at 420 nanometers (nm) was recorded. One unit of $\beta$-galactosidase activity represents that amount of the enzyme that hydrolyzes 1 nanomole (nM) of O-nitrophenyl-$\beta$-galoctoside per minute at 28°C.

d) Results

The $\beta$-galactosidase activity as assayed above for the JRY188 transformants carrying plasmid pJS12 was 198 units per $\mu$g of protein for cells grown to an $A_{600}$ density of 1.0.

In order to compare this enzyme activity level with a meaningful control, plasmid pJS12 was digested with HindIII and BamHI endonucleases thereby removing the SUC2 gene fragment from pJS12. The resulting sticky ends of HindIII and BamH1 digested pJS12 were filled in with deoxynucleotides and self-ligated to yield a fused mating factor $\alpha$-1 prepro-coding sequence-lacZ hybrid gene carried on the plasmid now referred to as pJS212. Plasmid pJS212 was transormed into Saccharomyces cerevisiae JRY188 and cultured as described previously. The $\beta$-galactosidase was isolated and the activity of the enzyme was measured. It was found that plasmid pJS212 which lacks the SUC2 gene fragment produced a $\beta$-galactosidase activity of only 77 units per $\mu$g of protein. Thus, the presence of the SUC2 gene fragment in the tripartite gene carried on plasmid pJS12 was capable of inducing a nearly 3-fold increase in enzyme activity over that produced with plasmid pJS212, i.e., a difference between 198 and 77 units per $\mu$g of protein, for the constructs respectively.

Example 2

Effect of NaCl on Protein Expression

In order to show the effect of NaCl on protein expression levels, the protocol set forth in Example 1 was repeated for both plasmids pJS12 and pJS212. However, the transformed cells were grown in the presence of 400 mM NaCl added to the culture medium. $\beta$-galactosidase activity was assayed as described above and for cells transformed with pJS12, the activity level was 1015 units per $\mu$g of protein. For those cells transformed with plasmid pJS212, the activity level was 331 units per $\mu$g of protein. It can be clearly seen that the use of NaCl increased expression levels of $\beta$-galactosidase when both plasmids were utilized. Interestingly, even the expression level from pJS212 (lacking the SUC2 gene fragment) increased as a result of the incorporation of NaCl in the culture medium. A similar increase was observed when KCl was used in place of NaCl.

Bibliography

The following publications which have been referred to in the instant specification are expressly incorporated herein by reference:

1. Bitter, G. et al, Proc. Natl. Acad. Sci. USA 81, 5330-5334 (1984)

2. Brake, A. et al, Proc. Natl. Acad. Sci. USA 81, 4642-4644 (1984)

3. Carlson, M. et al, Cell 28, 145-154 (1982)

4. Emr, S. et al, Proc. Natl. Acad. Sci. USA 80, 7080-7084 (1983).

5. Emr, S. et al, Mol. Cell. Biol. 4 (No. 11), 2347-2355 (1984)

6. Ito, H. et al, J. Bacteriol. 153, 163-168 (1982)

7. Kurjan, J. et al, Cell 30, 933-943 (1982)

8. Kurjan, J. et al, U.S. Patent 4,546,082 (1985)

9. Maniatis, T. et al, Molecular Cloning, A Laboratory Manual, Cold Spring Harbor Laboratory (1982)

10. Miller, J., Exp. Mol. Genet., 352 et seq (1972)

11. Schekman, et al, "The Molecular Biology of the Yeast Saccharomyces, Metabolism and Gene Expression", Cold Spring Harbor Press (1982)

12. Taussig, R. et al, Nucleic Acids Res. 11, 1943-1954 (1983)

## Claims

1. A DNA construct comprising the yeast mating factor $\alpha$-1 prepro-coding sequence tandemly linked to a 103 base pair region of the yeast SUC2 gene encoding the 34 amino-terminal amino acids of the invertase gene, and a structural gene encoding a polypeptide of choice in reading frame therewith.

2. A DNA expression vector comprising at least the yeast mating factor $\alpha$-1 prepro-coding sequence tandemly linked to a 103 base pair region of the yeast SUC2 gene encoding the 34 amino-terminal amino acids of the invertase gene, and a structural gene encoding a polypeptide of choice in reading frame therewith.

3. A yeast strain transformed with an expression vector comprising at least the yeast mating factor $\alpha$-1 prepro-coding sequence tandemly linked to a 103 base pair region of the yeast SUC2 gene encoding the 34 amino-terminal amino acids of the invertase gene, and a structural gene encoding a polypeptide of choice in reading frame therewith.

4. Plasmid pJS12.

5. A method for increasing the level of expression of a gene encoding a polypeptide of choice comprising culturing a yeast cell containing said gene in the presence of one or more chloride salts of alkali metals or alkaline-earth metals.

6. The method of Claim 5 wherein said chloride salt is sodium chloride or potassium chloride.

7. The method of Claim 6 wherein said chloride salt is present in a concentration of from about 100 millimolar to about 1 molar.

8. The method of Claim 7 wherein said chloride salt is present in a concentration of from about 200 millimolar to about 800 millimolar.

# FIG.I

```
                                                                -170
                                                                 •
                      -150                                      AGTG
                       •
CAAGAAAACCAAAAAGCAACAACAGGTTTTGGATAAGTACATATATAAGAGGGCCTTTTGTTCCCATCAAAAATGTTACTGTT
CTTACGATTCATTTACGATTCAAGAATAGTTCAAACAAGAAGATTACAAACTATCAATTTCATACACAATATAAACGACCAAA


                                            pst I                         50
                                                                          •
AGA ATG AGA ATT CCT TCA ATT TTT ACT GCA GTT TTA TTC GCA GCA TCC TCC GCA TTA GCT GCT
    met arg phe pro ser ile phe thr ala val leu phe ala ala ser ser ala leu ala ala
    1                                   10                                         20
                                                      100
                                                       •
CCA GTC AAC ACT ACA ACA GAA GAT GAA ACG GCA CAA ATT CCG GCT GAA GCT GTC ATC GGT TAC
pro val asn thr thr thr glu asp glu thr ala gln ile pro ala glu ala val ile gly tyr
                                     30                                        40
                              150
                               •
TCA GAT TTA GAA GGG GAT TTC GAT GTT GCT GTT TTG CCA TTT TCC AAC AGC ACA AAT AAC GGG
ser asp leu glu gly asp phe asp val ala val leu pro phe ser asn ser thr asn asn gly
                              50                                        60
              200
               •
TTA TTG TTT ATA AAT ACT ACT ATT GCC AGC ATT GCT GCT AAA GAA GAA GGG GTA TCT TTG GAT
leu leu phe ile asn thr thr ile ala ser ile ala ala lys glu glu gly val ser leu asp
              70                                                  80
250                   260 HindIII 270              280       290       300
                        •         •                 •         •         •
AAA AGA GAG          GCT GAA GCT TGG CAT TGG TTG CAA GTA AAA CCT GGC CAA CCA ATG TAC
lys arg glu          ala glu ala trp his trp leu gln leu lys pro gly gln pro met tyr
• • • • • • • • • • • • • • •                90                                  123
         SPACER 1                             α FACTOR 1
```

0 261 534

# FIG.I (continued)

```
          310               320        HindIII              340          350          360
          •                 •                                •            •            •
        AAG AGA GAA GCC GAA GCT GAA GCT TGG CAT TGG CTG CAA CTA AAG CCT GGC CAA CCA ATG TAC
        lys arg glu ala glu ala glu ala trp his trp leu gln leu lys pro gly gln pro met tyr
        .  .  .  .  .  .  .  .  .  .  .  .  . 111                                        123
                    SPACER 2                                    α FACTOR 2
      370                         380   HindIII 390            400          420          430
      •                           •                            •            •            •
      AAA AGA GAA GCC GAC GCT GAA GCT TGG CAT TGG CTG CAA CTA AAG CCC GGC CAA CCA ATG TAC
      lys arg glu ala asp ala glu ala trp his trp leu gln leu lys pro gly gln pro met tyr
      .  .  .  .  .  .  .  .  .  .  .  .  .       132                                    144
                    SPACER 3                                    α FACTOR 3
            440               450   HindIII    460            470          480          490
            •                 •                                •            •            •
        AAA AGA GAA GCC GAC GCT GAA GCT TGG CAT TGG TTG CAG TTA AAA CCC GGC CAA CCA ATG TAC
        lys arg glu ala asp ala glu ala trp his trp leu gln leu lys pro gly gln pro met tyr
        .  .  .  .  .  .  .  .  .  .  .  .  . 153                                        165
                    SPACER 4                                    α FACTOR 4
          500          510          520         '        SalI
          •            •            •
        TAA GCCCGACTGATAACAACAGTGTAGATGTAACAAAGTCGACTTTGTTCCCACTGTACTTTTAGCTCGTACAAAATACAAT
        stop


        ATACTTTTCATTTCTCCGTAAACAACCTGTTTTCCCATGTAATATCCTTTTCTATTTTTCGTTTCGTTACCAACTTTACACAT


        ACTTTATATAGCTAT
```

0 261 534

# FIG.2

```
ala phe leu phe leu leu ala gly phe ala ala lys ile ser ala ser met
GCT TTC CTT TTC CTT TTG GCT GGT TTT GCA GCC AAA ATA TCT GCA TCA ATG

thr asn glu thr ser asp arg pro leu val his phe thr pro asn lys gly
ACA AAC GAA ACT AGC GAT AGA CCT TTG GTC CAC TTC ACA CCC AAC AAG GCC
```

# FIG.3

## SCHEME FOR CONSTRUCTION OF MFα1-LAC Z FUSED GENE